# FASCICULE DE BREVET EUROPEEN

(11) **EP 1 007 524 B1**
(45) Date de publication et mention de la délivrance du brevet: **12.11.2003**
(21) Numéro de dépôt: 98942759.6
(22) Date de dépôt: 20.08.1998
(51) Int. Cl.: C07D 413/12, A61K 31/40, C07D 417/12, C07D 403/12, C07D 401/12, C07D 409/12

(54) **DERIVES D'INDOLE COMME AGONISTES DE 5-HT1B ET 5-HT1D**
INDOLDERIVATE ALS 5-HT1B UND 5-HT1D AGONISTEN
INDOLE DERIVATIVES AS 5-HT1B AND 5-HT1D AGONISTS

(30) Priorité: 25.08.1997 FR 9710606
(43) Date de publication de la demande: 14.06.2000
(73) Titulaire: PIERRE FABRE MEDICAMENT, 92100 Boulogne (FR)
(72) Inventeur: PEREZ, Michel, F-81100 Castres (FR); HALAZY, Serge, F-81090 Lagarrigue (FR)
(74) Mandataire: Ahner, Francis
(86) Numéro de dépôt international: FR9801826
(87) Numéro de publication internationale: WO99010344

(56) Documents cités:
- WO-A-95/14004
- FR-A- 2 699 918

## Description

La présente invention se rapporte à de nouveaux composés hétérocycliques dérivés de pipérazines indoliques ainsi qu'à leur procédé de préparation, les compositions pharmaceutiques les contenant et leur utilisation comme médicament.

La sérotonine ou 5-hydroxytryptamine (5-HT) joue un rôle important tant au niveau du système nerveux qu'au niveau cardiovasculaire. Des récepteurs sérotoninergiques ont été identifiés que ce soit au niveau central ou périphérique. Il est généralement admis que la sérotonine peut jouer un rôle important dans divers types de conditions pathologiques tels que
- certains désordres psychiatriques comme l'anxiété, la dépression, l'agressivité, les attaques de panique, les désordres compulsifs obsessionnels, la schizophrénie, la tendance au suicide,
- certains désordres neurodégénératifs comme la maladie d'Alzheimer, le Parkinsonisme,
- la migraine, les céphalées et
- les troubles liés à l'alcoolisme
(cf. E. Zifa, G. Fillion, Pharm. Reviews, **44**, 401, 1992 ; A. Moulignier, Rev. Neuro. (Paris) 150, 3-15, 1994 ; S. Langer, N. Brunello, G. Racagni, J. Mendlecvicz, "Serotonin receptor subtypes : pharmacological significance and clinical implications" Kargcr Ed. ; (1992) ; B.E. Léonard, Int. Clin. Psychopharmacology, 7, 13-21 (1992) ; R.W. Fuller ; J. Clin. Psychiatry, **53**, 36-45 (1992) ; D.G. Grahame-Smith, Int. Clin. Psychopharmacology, 6, suppl. 4, 6-13, (1992).

Les composés selon la présente invention sont des composés nouveaux ayant une très haute affinité et une très bonne sélectivité pour les récepteurs communément appelés 5-HT₁₋ₗᵢₖₑ et plus particulièrement pour les récepteurs appelés 5-HT_{1B} et 5-HT_{1D}.

Les médicaments, seuls ou en association avec d'autres agents thérapeutiques, incluant un ou plusieurs principes actifs de la présente invention trouvent leur emploi dans le traitement tant curatif que préventif des maladies liées au dysfonctionnement des récepteurs 5-HT₁₋ₗᵢₖₑ dont les récepteurs 5-HT_{1B} et 5-HT_{1D} à leur dérégulation, ou à des modifications de l'activité du ligand endogène qui est généralement la sérotonine.

Les composés de la présente invention sont des agonistes puissants, tant au niveau de leur affinité qu'au niveau de leur efficacité ou activité intrinsèque, et des agonistes sélectifs des récepteurs 5-HT_{1B} et 5-HT_{1D}. Les agonistes des récepteurs 5-HT₁₋ₗᵢₖₑ, et plus particulièrement des récepteurs 5-HT_{1B}, présentent une activité vasoconstrictrice sélective et trouvent leur utilisation dans le traitement de la migraine et des désordres vasospastiques (A. Doenicke et al., The Lancet, 1, 1309-1311, 1988; M.D. Ferrari, P.R. Saxena, Cephalalgia, 13, 151-165, 1993; S.J. Peroutka, Headache, 30, 5-11, 1990; M.A. Moskowitz, TiPS, 13, 307-311, 1992; W. Feniuk, P.P. Humphrey, M.S. Perren, H.E. Connor, E.T. Whalley, J. Neurol. 238, S57-S61, 1991; A.V. Deligonis, S.J. Peroutka, Headache, 31, 228-231, 1991),

Les composés de la présente invention, qui sont, pour la plupart, des agonistes puissants et sélectifs des récepteurs 5-HT_{1B} et 5-HT_{1D} trouvent donc plus particulièrement leur emploi dans le traitement curatif et prophylactique des crises de migraine classique, avec aura, de migraine commune, sans aura, l'algie vasculaire de la face, les céphalées chroniques vasculaires et des désordres vasospastiques.

L'état antérieur de la technique dans ce domaine est illustré notamment par :
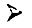 les demandes de brevets EP-A2- 0303507, WO 93/14087, WO 94/02460, WO 92/14708, et les brevets US 4,839,377, GB-A-2124210 et GB-A-2162532 qui décrivent des sulfonamides dérivées de tryptamines, dont le sumatriptan, comme antimigraineux.
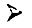 les demandes de brevet GB-A-2191488, GB-A-2185020 et GB-A-2168347 qui décrivent des alkylamides dérivées de tryptamine.
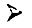 les demandes de brevet français FR-A-2 699 918 et FR-A-2 707 639 qui décrivent de nouveaux composés indoliques dérivés respectivement de pipérazines et d'arylamines comme ligands des récepteurs 5-HT_{1B} et 5-HT_{1D}.
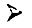 la demande de brevet d'invention FR-A-2 724 933 qui décrit de nouveaux éthers aromatiques dérivés d'indole comme ligands des récepteurs 5-HT_{1D}.
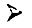 les demandes de brevet européen EP-A-0313397, EP-A-0486666, EP-A1-0494774, EP-A-0494774, EP-A2-0497512, EP-A1-0501568, EP-A-0464558, EP-A1-0548813 et les demandes internationales WO 92/13856 et 93/11106 qui décrivent des dérivés hétérocyliques dérivés de tryptamine comme agonistes des récepteurs 5-HT₁₋ₗᵢₖₑ.

La présente invention décrit une nouvelle classe de pipérazines dérivées d'amino-indole qui se distingue de tous les dérivés les plus proches de l'art antérieur par leur structure chimique originale et différente, mais aussi, par leur profil biologique et leur potentiel thérapeutique puisque de nombreux composés selon la présente invention présentent une très forte affinité et sélectivité pour les récepteurs 5-HT_{1B} et 5-HT_{1D}, une efficacité remarquable et un profil hémodynamique particulièrement intéressant. Les dérivés de la présente invention trouvent donc plus particulièrement leur utilité comme principes actifs de compositions médicamenteuses pour le traitement de la migraine et de divers troubles voisins.

La présente invention concerne des composés de formule générale (1). 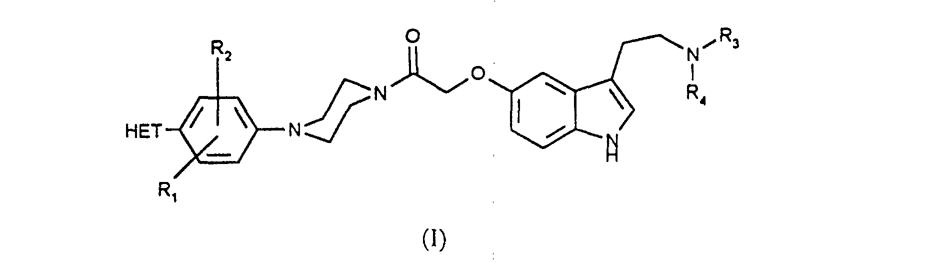 dans laquelle,
HET représente un hétérocycle choisi parmi 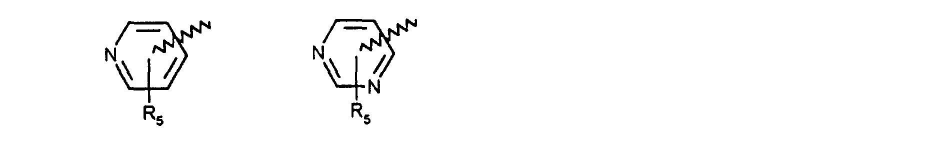 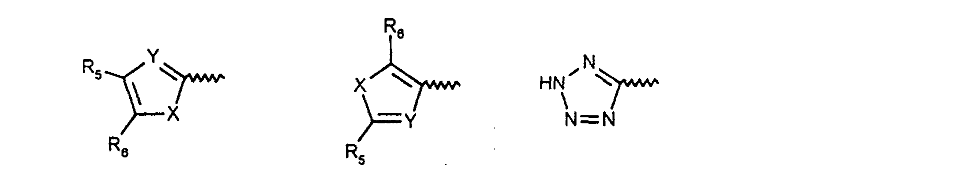 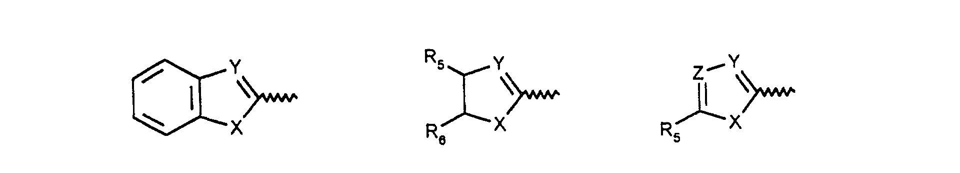
R₁ et R₂, identiques ou différents représentent un hydrogène, R'₁, CF₃, CH₂CF₃, C₆H₅, CH₂C₆H₅, OH, OR'₁, SH, SR'₁, Cl, F, Br, I, CN, NH₂, NHR'₁, NR'₁R'₂, NO₂, NH-NH₂, NH-NHR'₁, NHOH, NHCO₂R'₁, NHCONH₂, NHCONR'₁R'₂, NHSO₂R'₁, SO₂R'₁, SO₂NH₂, SO₂NHR'₁, COR'₁, CO₂R'₁, CONH₂, CONHR'₁, CONR'₁R'₂ pouvant être en position ortho ou meta sur le cycle aromatique,
R₃ et R₄, identiques ou différents représentent un hydrogène, un radical carboné linéaire ou ramifié comprenant de 1 à 6 atomes de carbone ou un reste benzyle ou phénétyle,
Y et Z, identiques ou différents représentent CH ou N,
X représente O, S ou NR₇,
R₅, R₆ et R₇, identiques ou différents, représentent un hydrogène, un reste alkyle linéaire ou ramifié comprenant de 1 à 6 atomes de carbone ou un reste phényle éventuellement substitué par un reste alkyle linéaire ou ramifié comprenant de 1 à 6 atomes de carbone, un halogène, CF₃, OCH₃, CN, ou NO₂.
R'₁ et R'₂, identiques ou différents, représentent un groupement alkyle linéaire ou ramifié comprenant de 1 à 6 atomes de carbone ou un reste phényle éventuellement substitué par un reste alkyle linéaire ou ramifié comprenant de 1 à 6 atomes de carbone, Cl, Br, F, I, OCH₃, OH, NO₂, SCH₃,
ainsi que leurs sels, et solvats acceptables pour l'usage thérapeutique.

Parmi les composés de formule générale (I) faisant partie de la présente invention, une classe de composés particulièrement appréciée correspond aux composés de formule générale (I) dans laquelle R₁, R₂, R₃ et R₄ représentent chacun un hydrogène.

Une autre classe particulièrement appréciée de composés faisant partie de la présente invention correspond aux composés de formule générale (I) dans laquelle HET représente un reste pyridyle ou pyrimidyle.

Une troisième classe particulièrement appréciée de composés faisant partie de la présente invention correspond aux composés de formule générale (I) dans laquelle HET représente un hétérocycle à 5 chaînons contenant de 1 à 3 hétéroatomes choisis parmi O, S ou N.

Parmi les composés de formule générale (I) à l'état de sels acceptables pour l'usage thérapeutique, on préfère les sels formés par addition avec des acides minéraux choisis parmi les chlorhydrates, les bromhydrates, les sulfates, les fumarates, les maléates, méthanesulfonates et succinates.

D'autres sels peuvent être utiles dans la préparation des composés de formule (I), par exemple les adduits avec le sulfate de créatinine.

La présente invention concerne également un procédé de préparation des composés de formule générale (I) qui consiste en la condensation d'une pipérazine aromatique de formule générale (II) 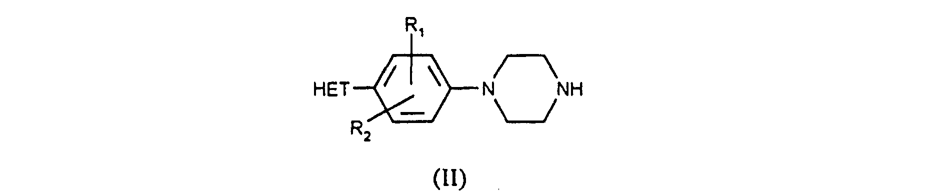 dans laquelle HET, R₁ et R₂ sont définis comme dans la formule générale (I) avec un acide carboxylique ou un dérivé d'acide carboxylique de formule générale (III) 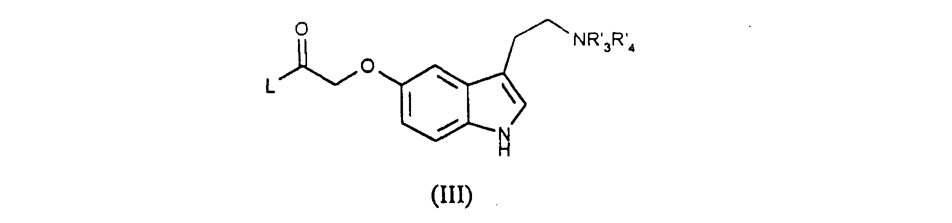 dans laquelle R'₃ et R'₄ sont identiques respectivement à R₃ et R₄ qui sont définis comme dans la formule générale (I), ou R'₃ et R'₄ sont des précurseurs ou groupes protecteurs de R₃ et R₄ qui seront transformés en R₃ et R₄ à la suite de la condensation de (II) avec (III), et L représente OH, Cl, O-alkyle ou le groupe -C(=O)L qui représente la forme activée d'un acide carboxylique propice à la formation d'une amide par réaction avec une amine.

Une variante particulièrement appréciée du procédé de préparation de l'invention met en oeuvre une amine de formule (II) et un composé de formule (III) dans laquelle L représente Cl, en présence d'une base organique ou inorganique telle que la pyridine, la DMAP, le DBU, K₂CO₃, Cs₂CO₃ ou Na₂CO₃ dans un solvant anhydre aprotique polaire tel que le THF, la DME, le dichlorométhane, à une température comprise entre - 20°C et 40°C.

Une deuxième variante particulièrement appréciée du procédé de préparation de l'invention met en oeuvre la condensation d'une amine de formule (II) avec un composé de formule (III) dans laquelle L représente OH, en présence d'une amine tertiaire telle que la triéthylamine, la diisopropyléthylamine, la pyridine, la DMAP, la N-méthylmorpholine, dans un solvant aprotique polaire tel que le THF, le dichlorométhane, le DCE, l'acétate d'éthyle, le chloroforme, le DMF, par réaction avec un agent activant tel que l'EDC, le DCC, le BOP, le PyBOP, à une température comprise entre -10 et 35°C.

Une méthode particulièrement appréciée dans le cadre de cette deuxième variante consiste à traiter le composé de formule (III) dans laquelle L représente OH avec le chloroformate d'éthyle en présence d'une base telle que, par exemple, une amine tertiaire comme la N-méthylmorpholine, dans un solvant aprotique polaire comme le dichlorométhane, le dichloroéthane, le THF et le DME, à une température comprise entre - 20°C et 0°C suivi de l'addition de l'amine de formule générale (II).

Dans le cas particulier des composés de formule (I) dans laquelle R₃ et R₄ représentent un hydrogène, la méthode de synthèse consiste à condenser une pipérazine hétéroaromatique de formule générale (II) avec un acide carboxylique ou un dérivé de cet acide carboxylique de formule générale (III) dans laquelle R'₃ représente un hydrogène et R'₄ représente le groupe protecteur t-butoxy-carbonyle selon les méthodes et techniques décrites précédemment et ensuite à hydrolyser le groupe protecteur t-butoxy-carbonyle en milieu acide, à l'aide par exemple de l'acide chlorhydrique ou de l'acide trifluoroacétique.

Un autre procédé de préparation des composés de formule générale (I) faisant partie de la présente invention, consiste à transformer une arylpipérazide dérivée de tryptamine de formule générale (IV) 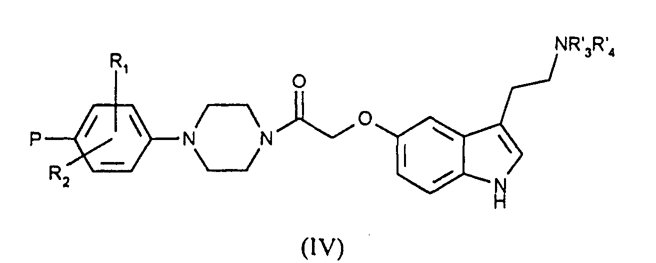 dans laquelle R₁ et R₂, R'₃ et R'₄ sont définis comme précédemment et P représente OSO₂CF₃, Br, I ou CN en un composé de formule générale (1) par diverses méthodes et techniques qui dépendront essentiellement de la nature de P dans le précurseur (IV) et de la nature de HET dans le composé de formule générale (I).

C'est ainsi que les composés de formule générale (IV) dans laquelle P représente I, Br ou OSO₂CF₃ peuvent être transformés en composés de formule générale (I) par condensation avec un acide boronique de formule HET-B(OH)₂ en présence de palladium selon la méthode bien connue de l'homme de métier dite « couplage de Suzuki ».

Les composés de formule générale (IV) dans laquelle P représente CN sont utilisés comme précurseurs de divers composés de formule générale (I) comme explicité dans le schéma suivant :

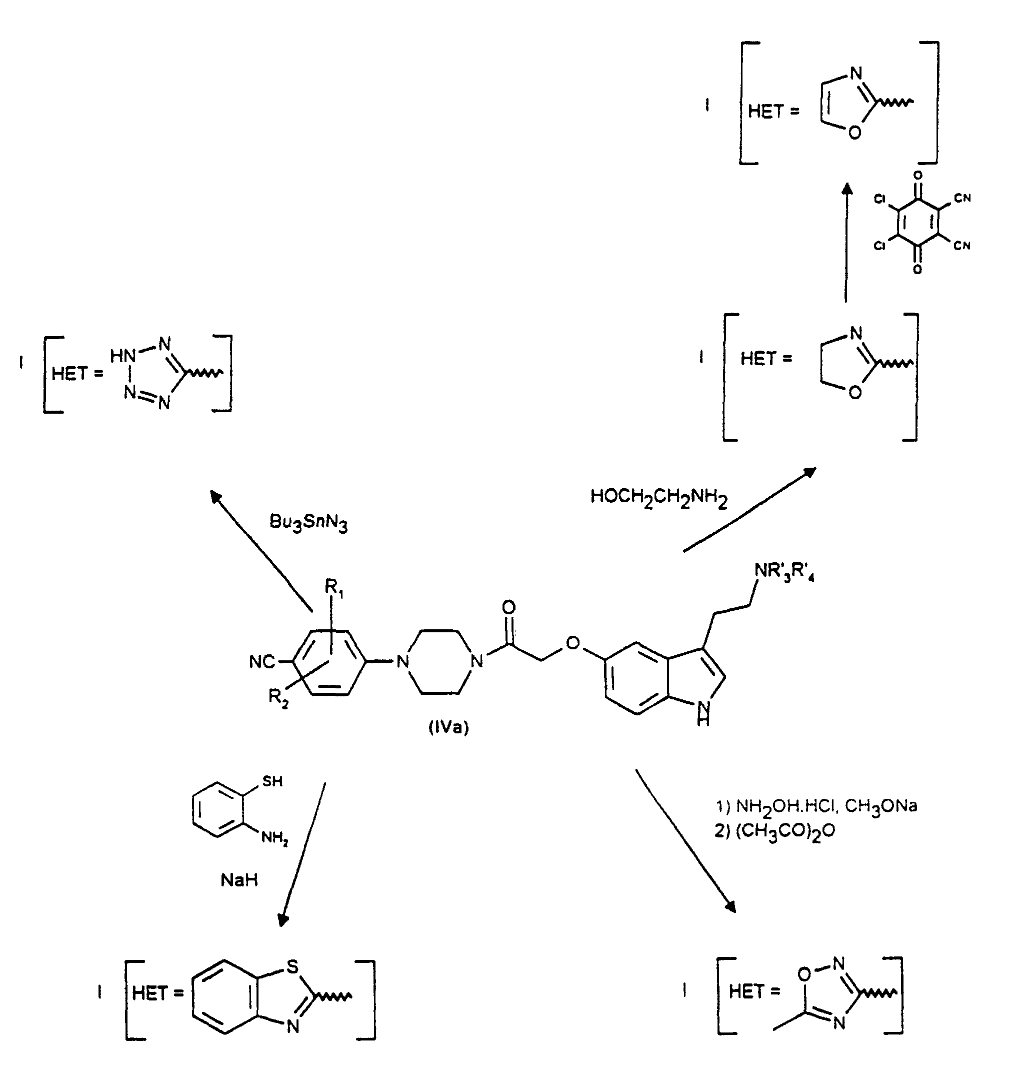

Il est bien entendu que, dans le cas où R'₃ et R'₄ sont différents de R₃ et R₄, les transformations indiquées dans ce schéma sous-entendent la mise en oeuvre de réactions complémentaires pour transformer R'₃, R'₄ en R₃, R₄. C'est ainsi que la préparation des composés de formule générale (I) dans laquelle R₃ et R₄ représentent un hydrogène par les méthodes décrites dans le schéma ci-dessus mettant en oeuvre un précurseur de formule générale (IV) dans laquelle R'₃ = H et R'₄ = COO^{t}Bu et une réaction complémentaire destinée à restaurer l'amine primaire telle que l'utilisation d'un acide comme l'acide chlorhydrique ou l'acide trifluoroacétique.

Les pipérazines aromatiques de formule générale (II) sont préparées par différentes méthodes et techniques bien connues de l'homme de l'art pour préparer des arylpipérazines. D'une manière générale, ces pipérazines intermédiaires sont préparées à partir d'un précurseur de formule générale (V) 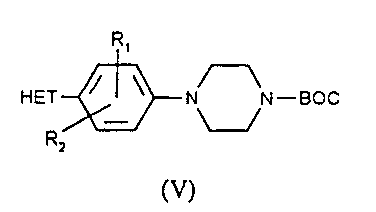 dans laquelle HET, R₁ et R₂ sont décrits comme précédemment, par réaction avec un acide tel que l'acide chlorhydrique ou préférentiellement l'acide trifluoroacétique.

Les intermédiaires de formule (V) sont accessibles à partir d'intermédiaires de formule générale (VI) 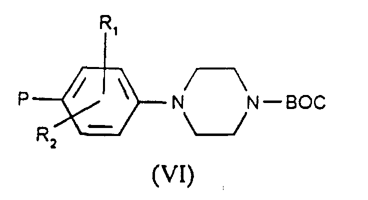 dans laquelle P est décrit comme précédemment.

La transformation des arylpipérazines de formule (VI) en arylpipérazines de formule (V) est réalisée par les différentes méthodes et techniques décrites précédemment pour la transformation des intermédiaires de formule générale (IV) en composés de formule (I).

Dans le cas particulier où, dans la formule générale (V), au moins un des substituants R₁ ou R₂ est un électroattracteur, par exemple NO₂, CF₃, CN ou CO₂R, attaché en position ortho sur le cycle aromatique par rapport au reste pipérazine, une méthode alternative pour obtenir les arylpipérazines de formule (V) consiste à condenser la N-BOC-pipérazine avec un électrophile de formule générale (VII). 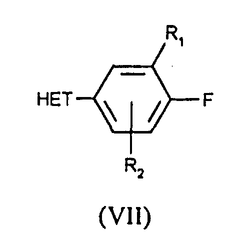 dans laquelle HET et R₂ sont définis comme précédemment et R₁ est un substituant électroattracteur, en présence d'une base organique ou inorganique, à une température comprise entre 20 et 100°C.

Les intermédiaires de formule générale (II) peuvent également être préparés à partir d'anilines de formule générale (VIII) 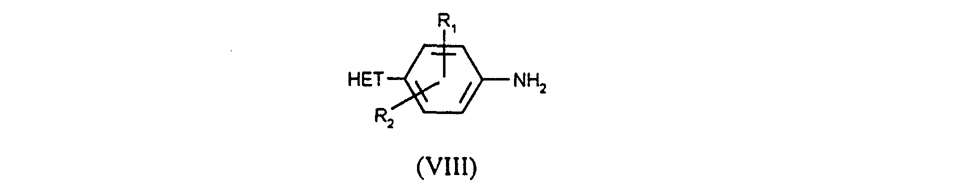 dans laquelle HET, R₁ et R₂ sont définis comme dans la formule générale I, après réaction avec des électrophiles de formule (IX) ou (X) 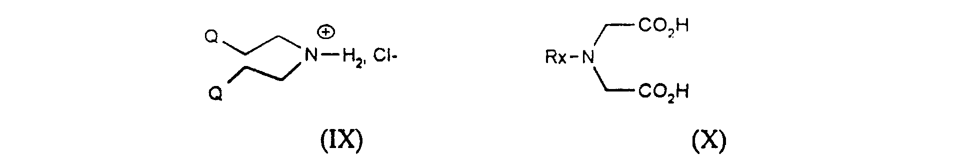 dans lesquelles Q représente un chlore, un brome, un iode, un tosylate ou un mésylate et Rₓ représente un groupe protecteur d'une amine, par exemple un t-butoxy-carbonyle, qui sera déprotégée ultérieurement. La condensation d'anilines de formule générale (VIII) avec des électrophiles de formule générale (IX) est réalisée préférentiellement dans un solvant anhydre polaire tel que le DMF, l'acétonitrile, le THF, le butanol, le t-butanol ou le DMSO, généralement à la température de reflux du solvant utilisé, en présence d'une base organique ou inorganique telle qu'un carbonate de potassium, de sodium ou de calcium.

Les intermédiaires de formule générale (II) peuvent également être préparés par condensation d'anilines de formule générale (VIII) avec des dérivés d'aminodiacides de formule (X), en présence d'anhydride acétique, suivi de la réduction de la dicétopipérazide intermédiaire ainsi formée avec, par exemple, un borane et enfin coupure du groupement protecteur, par exemple en milieu acide s'il s'agit d'un reste t-butoxy-carbonyle.

Doivent également être considérées comme faisant partie de la présente invention toutes les méthodes qui permettent de transformer un dérivé de formule (I) en un autre dérivé de formule (1) dans laquelle au moins un des substituants HET, R₁, R₂, R₃ ou R₄ est différent, par les techniques et méthodes bien connues de l'homme de l'art.

C'est ainsi et à titre d'exemple que les dérivés de formule générale (I) dans lesquels R₁ représente un groupe NO₂ peuvent être transformés en dérivés de formule (I) dans lesquels R₁ représente NH₂, par les méthodes et techniques bien connues pour ce type de réduction telles que décrites par exemple dans Comprehensive Organic Transformation, R.C. Larock, V.C.H., p. 412 1989, parmi lesquelles on peut citer l'hydrogénation atmosphérique catalysée par le palladium sur charbon, l'utilisation de SnCl₂ ou de zinc ou encore le catalyseur au rhodium en présence d'hydrazine. Les composés de formule (I) dans lesquelles R₁ représente NH₂ peuvent aussi être transformés en dérivés de formule (I) dans lesquels R₁ représente NR₈R₉, NHCO₂R₈, NHCOR₈R₉, NHSO₂R₈, par les méthodes et techniques bien connues de l'homme de métier pour transformer une amine aromatique en amide, carbamate, urée ou sulfonamide.

Dans le cadre de cette invention, il faut également considérer la préparation de composés de formule (I) à partir d'autres composés de formule (I) qui se distinguent par la nature du résidu HET. C'est ainsi que les dérivés de formule générale (I) dans laquelle HET représente un reste tétrazole peuvent être transformés en dérivés de formule (I) dans laquelle HET représente un oxadiazole suivant le schéma ci-dessous :

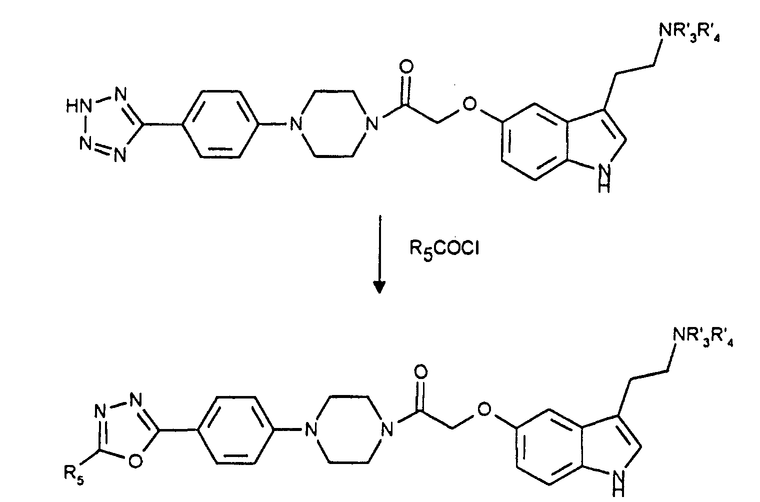

On comprendra que dans certaines réactions ou suites de réactions chimiques qui conduisent à la préparation de composés de formule générale (I) il est nécessaire ou souhaitable de protéger des groupes sensibles éventuels dans les intermédiaires de synthèse afin d'éviter des réactions secondaires indésirables. Ceci peut être réalisé par l'utilisation, i.e. l'introduction et la déprotection, des groupes protecteurs conventionnels tels que ceux décrits dans "Protective Groups in Organic Synthesis", T.W. Greene, John Wiley & Sons, 1981, et "Protecting Groups", P.J. Kocienski, Thieme Verlag, 1994. Les groupes protecteurs appropriés seront donc introduits et enlevés lors de l'étape la plus appropriée pour ce faire, en utilisant les méthodes et techniques décrites dans les références citées précédemment.

Lorsque l'on désire isoler un composé selon l'invention à l'état de sel, par exemple de sel par addition avec un acide, on peut y parvenir en traitant la base libre de formule générale (I) par un acide approprié de préférence en quantité équivalente.

La présente invention concerne également les composés de formule (I) pour leur application en tant que substances thérapeutiquement actives.

La présente invention concerne plus particulièrement les composés de formule (I) pour le traitement ou la prévention des désordres liés à la sérotonine, pour le traitement ou la prévention de la migraine, de l'algie vasculaire de la face, des céphalées chroniques vasculaires, pour le traitement ou la prévention de la dépression, des désordres compulsifs obsessionnels, de la boulimie, de l'agressivité, de l'alcoolisme, des nausées, du dysfonctionnement sexuel, du comportement asocial, de l'anxiété, de la spasticité, des maladies d'Alzheimer et de Parkinson.

La présente invention concerne également les compositions pharmaceutiques contenant à titre d'ingrédient actif au moins un composé de formule (I) et un excipient pharmaceutiquement acceptable.

Les compositions selon l'invention peuvent être employés par voie orale, nasale, parentérale, rectale ou topique.

Comme compositions solides pour administration orale, peuvent être utilisés des comprimés, des pilules, des poudres sous forme de capsules de gélatine ou de cachets, des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée ; elles sont généralement comprises entre 0,001 g et 1 g, de préférence comprises entre 0,005 g et 0,25 g par jour, de préférence par voie orale pour un adulte avec des doses unitaires allant de 0,1 mg à 500 mg de substance active, de préférence de 1 mg à 50 mg.

D'une façon générale, le médecin déterminera la posologie appropriée en fonction de l'âge, du poids et de tous les autres facteurs propres au sujet à traiter. Les exemples qui suivent illustrent l'invention sans toutefois en limiter la portée.

### EXEMPLE 1

### Fumarate du 2-[3-(2-amino-éthyl)-1H-indol-5-yloxy]-1-{4-[4-(5-phényl-[1,3,4]oxadiazol-2-yl)-phényl]-pipérazin-1-yl}-éthanone

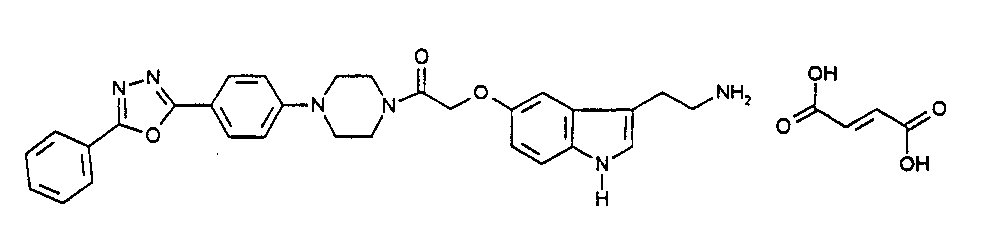

### 1A) N-terbutoxycarbonyl-4-(4-cyanophényl)-pipérazine

La 1-(4-cyanophényl)-pipérazine (5,0 g; 26,7 mmol) en solution dans le dichlorométhane (100 ml) en présence de triéthylamine (5,6 ml; 40,0 mmol) est traité par le diterbutyldicarbonate (6,4 g; 29,3 mmol). Après 1 h d'agitation à température ambiante le milieu est dilué au dichlorométhane et lavé à l'eau. La phase organique est séchée sur sulfate de sodium, filtrée et évaporée à sec pour conduire à l'intermédiaire **1A** (7,7 g; 100 %) sous la forme d'un solide beige.

### 1B) N-terbutoxycarbonyl-4-[4-(2H-tétrazol-5-yl)-phényl]-pipérazine

L'intermédiaire **1A** (6,0 g; 20,8 mmol) en solution dans le xylène (76 ml), sous azote, est traité par l'azidure de triméthyl étain (6,25 g; 31,3 mmol). Après 24 h d'agitation à 110°C le milieu réactionnel est ramené à température ambiante et le précipité formé est filtré sur un verre fritté puis lavé au toluène et à l'éther de pétrole. Ce solide est purifié par chromatographie sur colonne de gel de silice éluée par un mélange dichlorométhane/méthanol/ammonique dans les proportions 80/19/1 pour conduire au composé **1B** (5,75 g; 87 %) sous la forme d'un solide blanc.

### 1C) 1-[4-(5-phényl-[1,3,4]oxadiazol-2-yl)-phényl]-pipérazine

Le composé **1B** (1,5 g; 4,54 mmol) en solution dans la pyridine (15 ml) est chauffé à 60°C, sous azote, pendant 10 mn. Le chlorure de benzoyle (1,3 ml; 11,35 mmol) est alors additionné et le mélange est chauffé au reflux pendant 3 h. Pour compléter la réaction (0,5 ml; 4,54 mmol) de chlorure de benzoyle sont de nouveau additionnés et le mélange est chauffé 1 h de plus au reflux.

Le milieu réactionnel est ensuite ramené à température ambiante, dilué à l'acétate d'éthyle, lavé successivement à l'eau, par une solution saturée de sulfate de cuivre, à l'eau et enfin par une solution saturée de chlorure de sodium. La phase organique est séchée sur sulfate de sodium, filtrée et évaporée à sec.

Le solide beige obtenu est repris dans le toluène (46 ml) et traité par l'acide trifluoroacétique (6 ml). Après 1 h d'agitation à température ambiante le milieu est dilué à l'acétate d'éthyle, lavé à la soude (2N) puis à l'eau et enfin par une solution saturée de chlorure de sodium. La phase organique est séchée sur sulfate de sodium, filtrée et évaporée à sec. Le sirop obtenu est chromagraphié sur colonne de gel de silice éluée par un mélange dichlorométhane/méthanol/ammonique dans les proportions 95/4,5/0,5 pour conduire au composé **1C** pur (800 mg; 57 %).

### 1) Fumarate du 2-[3-(2-amino-éthyl)-1H-indol-5-yloxy]-1-{4-[4-(5-phényl-[1,3,4]oxadiazol-2-yl)-phényl]-pipérazin-1-yl}-éthanone

L'acide 3-[2-(N-terbutoxycarbonyl)amino-éthyl]-*1H*-indol-5-yloxyacétique (763 mg; 2,28 mmol), ou acide de SerBOC (Bioorg. Med. Chem. Lett., 5, 1995, 663-666), en solution dans le dichlorométhane (9 ml), en présence de N-méthylmorpholine (0,27 ml; 2,50 mmol), est traité à - 10°C sous azote par le chloroformate d'éthyle (0,24 ml; 2,50 mmol). Après 10 mn d'agitation à - 10°C l'intermédiaire **1C** (769 mg; 2,50 mmol) est additionné et le mélange est agité de -10°C à température ambiante pendant 2 h. Le milieu est dilué à l'acétate d'éthyle, lavé successivement à l'eau, par une solution de bicarbonate de sodium et enfin par une solution saturée de chlorure de sodium. La phase organique est séchée sur sulfate de sodium, filtrée et évaporée à sec.

Le sirop obtenu est chromagraphié sur colonne de gel de silice éluée par un mélange dichlorométhane/acétone dans des proportions allant de 8/1 à 4/1 pour conduire au produit de couplage (1,19 g; 84 %). Ce composé est repris dans le toluène (30 ml) et traité par l'acide trifluoroacétique (3,9 ml) à température ambiante, pendant 1 h. Le milieu est dilué à l'acétate d'éthyle puis basifié par addition de soude 2N.

Le précipité formé est isolé par filtration puis lavé à l'eau, à l'acétate d'éthyle et à l'éther. Ce solide est purifié par chromatographie sur colonne de gel de silice éluée par un mélange dichlorométhane/méthanol/ammoniaque dans les proportions 90/9/1 pour conduire au produit attendu sous forme de base (746 mg; 75 %).

Ce produit est repris dans le méthanol puis salifié par addition d'acide fumarique (165 mg; 1,43 mmol) pour donner un solide blanc.
RMN ¹H DMSO-d6 (ppm) : 2,92 t, 2H; 3,03 t, 2H; 3,33-3.50 m, 4H; 3,60-3,75 m, 4H; 4,83 s, 2H; 6,41 s, 2H; 6,80 dd, 1H; 7,10-7.20 m, 4H; 7,26 d, 1H; 7,59-7,65 m, 3H; 7,96 d, 2H; 8,08-8,12 m, 2H; 10,81 s, 1H

| Analyse élémentaire (C₃₄H₃₄N₆O₇, 1,5 H₂O) | | | |
|---|---|---|---|
| % Calculés | C 61,35 | H 5,60 | N 12,62 |
| % Trouvés | C 61,14 | H 5,43 | N 12,70 |

Point de fusion : 170°C
Spectre de masse : m/z 523 (MH⁺)

### EXEMPLE 2

### Fumarate du 2-[3-(2-amino-éthyl)-1H-indol-5-yloxy]-1-{4-[4-(5-o-tolyl-[1,3,4]oxadiazol-2-yl)-phényl]-pipérazin-1-yl}-éthanone

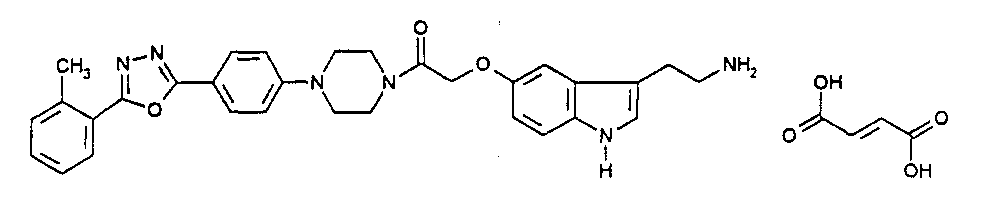

Le composé **2** est préparé à partir de l'intermédiaire **1B** (1,0 g; 3,03 mmol), de chlorure de *o*-toluoyle (0,99 ml; 7,55 mmol) et d'acide de SerBOC (576 mg; 1,72 mmol) selon la procédure décrite pour la préparation de l'exemple 1.
RMN ¹H DMSO-d6 (ppm) : 2,68 s, 3H; 2,93 t, 2H; 3,04 t, 2H; 3,30-3,50 m, 4H; 3,60-3,75 m, 4H; 4,83 s, 2H; 6,42 s, 2H; 6,80 d, 1H; 7,11-7,20 m, 4H; 7,26 d, 1H; 7,40-7,53 m, 3H; 7,95 d, 2H; 8,04 d, 1H; 10,83 s, 1H

| Analyse élémentaire C₃₅H₃₆N₆O₇; 1,5H₂O | | | |
|---|---|---|---|
| % Calculés | C 61,85 | H 5,78 | N 12,36 |
| % Trouvés | C 62,07 | H 5,74 | N 12,26 |

Point de fusion : 123°C
Spectre de masse : m/z 537 (MH⁺)

### EXEMPLE 3

### Fumarate du 2-[3-(2-amino-éthyl)-1H-indol-5-yloxy]-1-{4-[4-(5-p-tolyl-[1,3,4]oxadiazol-2-yl)-phényl]-piperazin-1-yl}-éthanone

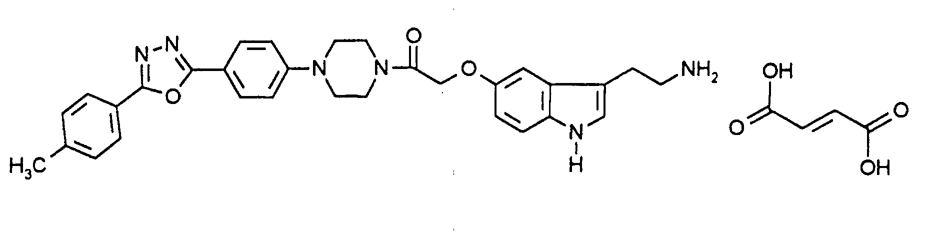

Le composé **3** est préparé à partir de l'intermédiaire **1B** (900 mg; 2,72 mmol), de chlorure de *p*-toluoyle (1,0 ml; 7,6 mmol) et d'acide SerBOC (186 mg; 0,55 mmol) selon la procédure décrite pour la préparation de l'exemple 1.
RMN ¹H DMSO-d6 (ppm) : 2,41 s, 3H; 2,94 t, 2H; 3,03 t, 2H; 3,32-3,48 m, 4H; 3,65-3,71 m, 4H; 4,84 s, 2H; 6,42 s, 2H; 6,80 dd, 1H; 7,10-7,18 m, 3H; 7,20 s, 1H; 7,27 d, 1H; 7,43 d, 2H; 7,98 dd, 4H; 10,83 s, 1H

| Analyse élémentaire C₃₅H₃₆N₆O₇, 2H₂O | | | |
|---|---|---|---|
| % Calculés | C 61,04 | H 5,85 | N 12,20 |
| % Trouvés | C 60,84 | H 5,60 | N 12,07 |

Spectre de masse : m/z 537 (MH⁺)
Point de fusion : 135°C

### EXEMPLE 4

### Fumarate du 2-[3-(2-amino-éthyl)-1H-indol-5-yloxy]-1-{4-[4-(5-méthyl-[1,3,4]oxadiazol-2-yl)-phényl]-pipérazin-1-yl}-éthanone

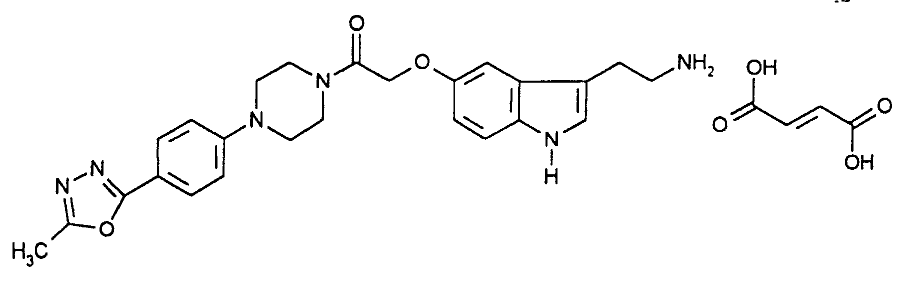

Le composé **4** est préparé à partir de l'intermédiaire **1B** (205 mg; 0,62 mmol) du chlorure d'acétyle (0,16 ml; 2,23 mmol) et de l'acide de SerBOC (84 mg; 0,25 mmol) selon la procédure décrite pour la préparation de l'exemple **1**.
RMN ¹H DMSO-d6 (ppm) : 2,55 s, 3H; 2,90-3,10 m, 4H; 3,30-3,40 m, 4H; 3,68 large s, 4H; 4,83 s, 2H; 6,46 s, 2H; 6,80 d, 1H; 7,08-7,29 m, 5H; 10,86 s, 1H

| Analyse élémentaire C₂₉H₃₂N₆O₇, 1,6H₂O | | | |
|---|---|---|---|
| % Calculés | C 57,63 | H 5,87 | N 13,96 |
| % Trouvés | C 58,02 | H 5,80 | N 13,56 |

Point de fusion : 145-150°C

### EXEMPLE 5

### Fumarate du 2-[3-(2-amino-éthyl)-1H-indol-5-yloxy]-1-{4-[4-(4,5-dihydrooxazol-2-yl)-phényl]-pipérazin-1-yl}-éthanone

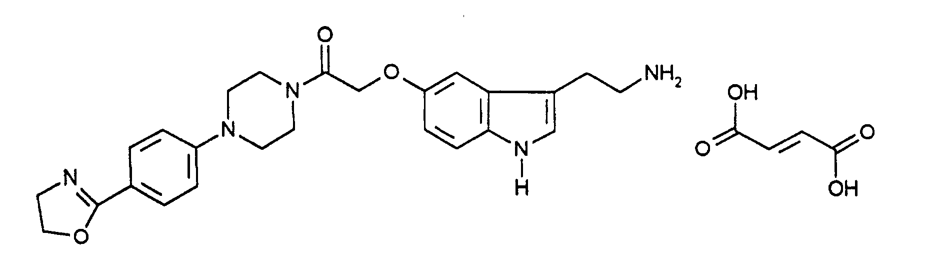

### 5A) N-terbutoxycarbonyl-4-[4-(4,5-dihydro-oxazol-2-yl)-phényl]-pipérazine

Un mélange d'éthanolamine (0,91 ml; 15,0 mmol), de glycérol (2,7 g), d'éthylène glycol (5,6 g) et de carbonate de potassium (2,1 g; 15,0 mmol) est chauffé à 105°C pendant 30 mn. L'intermédiaire **1A** (2,3 g; 8,04 mmol) est alors additionné et le mélange est chauffe 5 jours à 105°C. Le précipité formé est filtré sur un verre fritté et lavé à l'eau et à l'éther de pétrole pour conduire au composé **5A** (988 mg; 37 %) sous la forme d'un solide beige.

### 5B) 4-[4-(4,5-dihydro-oxazol-2-yl)-phényl]-pipérazine

Le composé **5A** (826 mg; 2,49 mmol) en solution dans le toluène (20 ml) est traité, à température ambiante, par l'acide trifluoroacétique (2,75 ml). Après 1 h, le milieu est évaporé à sec et coévaporé 3 fois au toluène. Le produit obtenu est repris dans le méthanol (20 ml) et la triéthylamine (8 ml), agité 15 mn à température ambiante et enfin évaporé à sec. Le solide obtenu est purifié par chromatographie sur colonne de gel de silice éluée par un mélange dichlorométhane/méthanol/ammoniaque dans les proportions 90/9/1 pour conduire à l'intermédiaire **5B**.

### 5) Fumarate du 2-[3-(2-amino-éthyl)-1H-indol-5-yloxy]-1-{4-[4-(4,5-dihydrooxazol-2-yl)-phényl]-pipérazin-1-yl}-éthanone

Le composé **5** est préparé à partir de l'intermédiaire **5B** (576 mg; 2,48 mmol) et de l'acide de SerBOC (694 mg; 2,07 mmol) selon la procédure décrite pour la préparation de l'exemple **1** à partir de **1C**.
RMN ¹H DMSO-d6 (ppm) : 2,94 t, 2H; 3,05 t, 2H; 3,25-3,38 m, 4H; 3,55-3,72 m, 4H; 3,89 t, 2H; 4,33 t, 2H; 4,82 s, 2H; 6,45 s, 2H; 6,80 dd, 1H; 7,00 d, 2H; 7,14 d, 1H; 7,19 d, 1H; 7,26 d, 1H; 7,71 d, 2H; 10,84 s, 1H

| Analyse élementaire C₂₉H₃₃N₅O₇; 2,9H₂O | | | |
|---|---|---|---|
| % Calculés | C 56,56 | H 6,35 | N 11,37 |
| % Trouvés | C 56,84 | H 6,06 | N 11,35 |

Point de fusion : 170°C
Spectre de masse : m/z 448 (MH')

### EXEMPLE 6

### Chlorhydrate du 2-[3-(2-amino-éthyl)-1H-indol-5-yloxy]-1-[4-(4-oxazol-2-yl-phényl)-pipérazin-1-yl]-éthanone

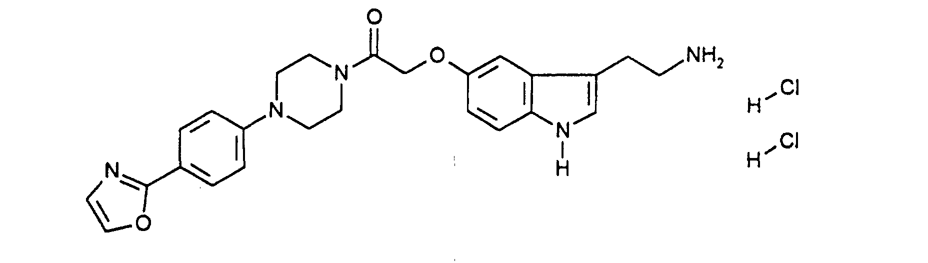

### 6A) 4-(4-oxazol-2-yl-phényl)-pipérazine

L'intermédiaire **5A** (597 mg; 1,80 mmol) en solution dans le xylène (8 ml) est traité par la 2,3-dichloro-5,6-dicyano-1,4-benzoquinone (450 mg; 1,98 mmol). Le mélange est chauffé pendant 50 mn au reflux puis évaporé à sec. Le solide noir obtenu est purifié par chromatographie sur colonne de gel de silice éluée par un mélange hexane/acétate d'éthyle dans le 3/1 pour donner un solide jaune (59 mg; 10 %). Ce composé est déprotégé selon la procédure décrite pour la préparation du composé **5B** à partir du composé **5A** pour conduire au sel de l'acide trifluoroacétique du composé **6A** sous la forme d'un solide beige (54 mg; 100 %).

### 6) Fumarate du 2-[3-(2-amino-éthyl)-1H-indol-5-yloxy]-1-[4-(4-oxazol-2-yl-phényl)-pipérazin-1-yl]-éthanone

Le composé **6** est préparé à partir de l'intermédiaire **6A** (54 mg; 0,16 mmol) et de l'acide de SerBOC (53 mg; 0,16 mmol) selon la procédure décrite pour la préparation de l'exemple **1** à partir de **1C**.
RMN ¹H DMSO-d6 (ppm) : 2,90-3,00 m, 2H; 3,00-3,10 m, 2H; 3,25-3,40 m, 4H; 3,59-3,74 m, 4H; 4,83 s, 2H; 6,81 dd, 1H; 7,08 d, 2H; 7,14 s, 1H; 7,21 s, 1H; 7,25-7,31 m, 2H; 7,83 d, 2H; 7,90 large s, 3H; 8,11 s, 1H, 10,85 s, 1H

| Analyse élémentaire C₂₅H₂₇N₅O₃; 2,2HCl; 3,5H₂O | | | |
|---|---|---|---|
| % Calculés | C 51,00 | H 6,20 | N 11,89 |
| % Trouvés | C 51,21 | H 5,82 | N 11,70 |

Point de fusion : 64-66°C (dégradation)
Spectre de masse : m/z 446 (MH⁺)

### EXEMPLE 7

### Fumarate du 2-[3-(2-amino-éthyl)-1H-indol-5-yloxy]-1-[4-(4-benzothiazol-2-yl-phényl)-pipérazin-1-yl]-éthanone

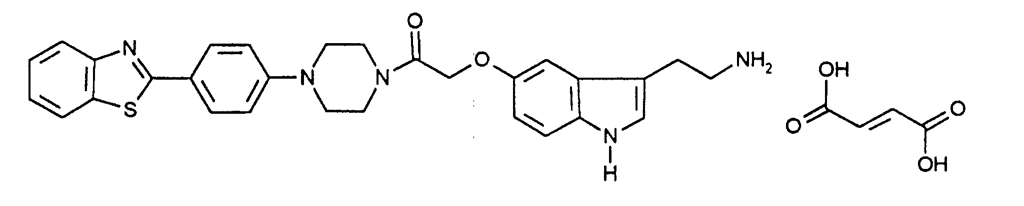

Le 2-aminothiophénol (0,32 ml; 3,0 mmol) en solution dans le tétrahydrofuranne (10 ml) est traité, sous azote et à température ambiante, par l'hydrure de sodium (60 % dans l'huile) (480 mg; 12,0 mmol).

Le milieu est porté à 60°C puis l'intermédiaire **1A** (861 mg; 3,0 mmol) est additionné. Le mélange est maintenu à 60°C pendant 3h30 puis refroidi à 5°C et neutralisé par addition de chlorure d'ammonium.

Le mélange est extrait à l'acétate d'éthyle puis au dichlorométhane. Les phases organiques sont rassemblées, séchées sur sulfate de sodium et évaporé à sec. Le sirop obtenu est purifié par chromatographie sur colonne de gel de silice éluée par un mélange dichlorométhane/acétate d'éthyle dans le 20/1 pour conduire au produit de condensation (760 mg; 64 %).

Le composé 7 est préparé à partir de l'intermédiaire précédent déprotégé puis condensé avec l'acide de SerBOC (170 mg; 0,51 mmol) selon la procédure décrite pour la préparation de l'exemple **1**.
RMN ¹H, DMSO-d6 (ppm) ; 2,95-3,04 m, 4H; 3,30-3,45 m, 4H; 3,68 large s, 4H; 4,84 s, 2H; 6,44 s, 2H; 6,80 dd, 1H; 7,08-7,29 m, 5H; 7,35-7,53 m, 2H; 7,92-7,98 m, 3H; 8,07 d, 1H; 10,85 s, 1H

| Analyse élémentaire C₂₉H₂₉N₅O₂S; 0,8C₄H₄O₄; 1,3H₂O | | | |
|---|---|---|---|
| % Calculés | C 61,48 | H 5,57 | N 11,09 |
| % Trouvés | C 61,02 | H 5,42 | B 10,73 |

Point de fusion : 156-160°C
Spectre de masse : m/z 512 (MH⁺)

### EXEMPLE 8

### Chlorhydrate de 2-[3-(2-amino-éthyl)-1H-indol-5-yloxy]-1-{4-[4-(2H-tétrazol-5-yl)-phényl]-pipérazin-1-yl}-éthanone

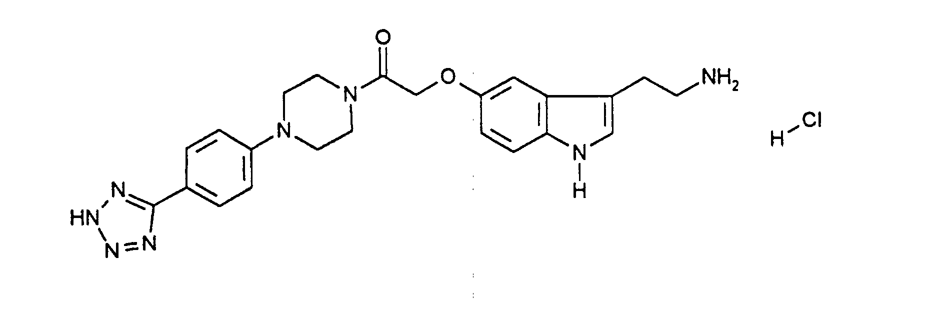

Le 4 - (4-{2-[3-(2-{N-terbutoycarbonyl}-amino-éthyl)- 1H-indol-5-yloxy] -acétyl}-pipérazin-1-yl)-benzonitrile (J. Med. Chem., 1995, 38, 3602-3607) (400 mg; 0,79 mmol) en solution dans le xylène (5 ml) est traité, sous azote, par l'azidure de triméthyl étain (310 mg; 1,50 mmol). Le mélange est chauffé 5 jours à 110°C puis le précipité formé est filtré sur verre fritté et lavé au toluène pour conduire à un solide crème.

Ce composé est purifié par chromatographie sur colonne de gel de silice éluée par un mélange dichlorométhane/méthanol/ammoniaque dans les proportions 85/14/1 pour conduire à une poudre jaune (350 mg; 81 %).

Cet intermédiaire est déprotégé selon les conditions décrites pour la préparation du composé **5B** à partir du composé **5A**, pour conduire au composé 8 (209 mg; 73 %) isolé sous forme de chlorhydrate.
RMN ¹H, DMSO-d6 (ppm) : 2,95-3,05 m, 4H; 3,38 large s, 4H; 3,67 large s, 4H; 4,82 s, 2H; 6,80 dd, 1H; 7,11-7,28 m, 5H; 7,80-7,95 m, 5H; 10,84 s, 1H
Point de fusion : 225-227°C
Spectre de masse : 447 (MH⁺)

### EXEMPLE 9

### Fumarate du 2-[3-(2-amino-éthyl)-1H-indol-5-yloxy]-1-[4-(4-pyridin-4-yl-phényl)-pipérazin-1-yl]-éthanone

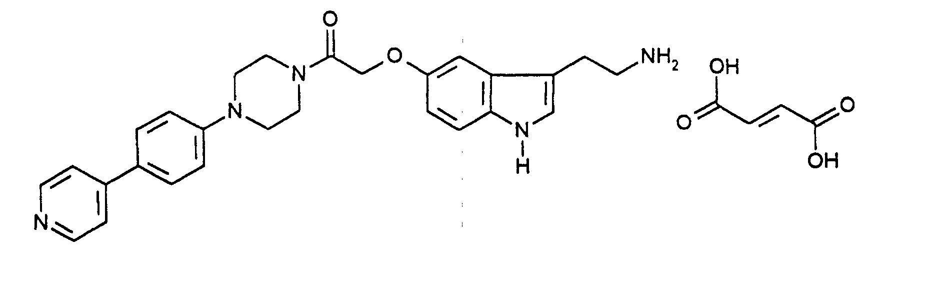

### 9A) 2-chloro-1-[(4-hydroxy-phényl)-pipérazin-1-yl]-éthanone

La 4-hydroxy-phényl-pipérazine (8 g; 44,9 mmol) en solution dans la méthyléthylcétone (43 ml), en présence de carbonate de potassium (15,5 g; 112 mmol) est traité à 0°C et goutte à goutte par le chlorure de chloracétyle (5,4 ml; 67,2 mmol).

Après 1 h 30 d'agitation à 0°C le milieu est dilué à l'acétate d'éthyle, filtré sur célite, lavé à l'eau puis par une solution de chlorure de sodium. La phase organique est séchée sur sulfate de sodium, filtrée et évaporée.

Le produit obtenu est purifié par chromatographie sur colonne de gel de silice éluée par un mélange dichlorométhane/méthanol/ammoniaque dans les proportions 95/4,5/0,5 pour conduire au composé **9A** (8,2 g; 72 %) sous la forme d'un solide beige.

### 9B) 2-chloro-1-[(4-trifluorosulfonate-phényl)-piperazin-1-yl]-éthanone

Le composé **9A** (7,8 g; 30,6 mmol) en solution dans le dichlorométhane (428 ml), en présence de triéthylamine (10,6 ml; 76,5 mml) est traité, sous azote et à 0°C, par l'anhydride triflique (7,7 ml; 45,9 mmol).

Après 1 h d'agitation de 0°C à température ambiante le milieu est dilué au dichlorométhane, lavé par une solution de bicarbonate de sodium, puis par une solution de chlorure de sodium.

La phase organique est séchée sur sulfate de sodium puis filtrée et évaporée à sec. Le produit obtenu est purifié par chromatographie sur colonne de gel de silice éluée par un mélange dichlorométhane/acétone dans les proportions 100/1 pour conduire au produit **9B** (10,8 g; 91 %).

### 9C) 2-[3-(2-{N-terbutoxycarbonyl-amino-éthyl)-1H-indol-5-yloxy]-1-[4-(4-trifluorosulfonate-phényl)-pipérazin-1-yl]-éthanone

Le 3-[2-(N-terbutoxycarbonyl)-amino-éthyl]-lH-indol-5-ol (J. Med. Chem., 1995, 38, 3602-3607) (3,5 g; 12,68 mmol) en solution dans la méthyléthylcétone (140 ml), en présence de carbonate de potassium (4,37; 31,7 mmol) et d'iodure de potassium (0,21 g; 1,26 mmol) est traité à température ambiante par l'intermédiaire **9B** (10,8 g; 27,9 mmol).

Le mélange est porté au reflux pendant 8 h puis ramené à température ambiante et agité pendant une nuit. Le milieu est dilué à l'acétate d'éthyle, lavé à l'eau puis par une solution de chlorure de sodium. La phase organique est séchée sur sulfate de sodium, filtrée et évaporée à sec.

Le produit obtenu est purifié par chromatographie sur colonne de gel de silice éluée par un mélange dichlorométhane/acétate d'éthyle dans les proportions 9/1 pour conduire au produit **9C** (3,87 g; 49 %).

### 9) 2-[3-(2-amino-éthyl)-1H-indol-5-yloxy]-1-[4-(4-pyridin-4-yl-phényl) pipérazin-1-yl]-éthanone

Un mélange de l'intermédiaire **9C** (600 mg; 0,96 mmol), de l'acide 4-pyridinyl-boronique (117 mg; 1,44 mmol), de chlorure de lithium (121 mg; 2,88 mmol) et de palladium tétrakis (155 mg; 0,134 mmol) dans un mélange de diméthoxyéthane (3,1 ml) et de carbonate de sodium 2M (1,3 ml) est chauffé à 105°C et sous azote pendant 23 h. Le milieu est dilué à l'acétate d'éthyle, lavé à la soude (2N) puis à l'eau et enfin par une solution de chlorure de sodium. La phase organique est séchée sur sulfate de sodium, filtrée et évaporée à sec.

Le produit obtenu est purifié par chromatographie sur colonne de gel de silice éluée par un mélange dichlorométhane/méthanol/ammoniaque dans les proportions 99/1/0,1 puis dans les proportions 90/9/1 pour conduire à un sirop (259 mg; 49 %).

Ce composé est déprotégé dans les conditions décrites pour la préparation du composé **5B** à partir du composé **5A** pour conduire au composé 9 (142 mg; 67 %) isolé sous forme de fumarate.
RMN ¹H DMSO-d6 (ppm) : 2,92 s, 2H; 3,03 s, 2H; 3,20-3,35 m, 4H; 3,66 d, 4H; 4,82 s, 2H; 6,41 s, 2H; 6,80 d, 1H; 7,08 d, 2H; 7,13 s, 1H; 7,18 s, 1H; 7,26 d, 1H; 7,50-7,65 m, 2H; 7,72 d, 2H; 8,54 s, 2H; 10,81 s, 1H

| Analyse élémentaire C₂₇H₂₉N₅O₂,1,1C₄H₄O₄; 1,65H₂O | | | |
|---|---|---|---|
| % Calculés | C 61,53 | H 6,03 | N 11,43 |
| % Trouvés | C 61,63 | H 5,85 | N 11,03 |

Point de fusion : 176°C
Spectre de masse : m/z : 456 (MH⁺)

### EXEMPLE 10

### Fumarate du 2-[3-(2-amino-éthyl)-1H-indol-5-yloxy]-1-[4-(4-thiophén-2-yl-phényl)-pipérazin-1-yl]-éthanone

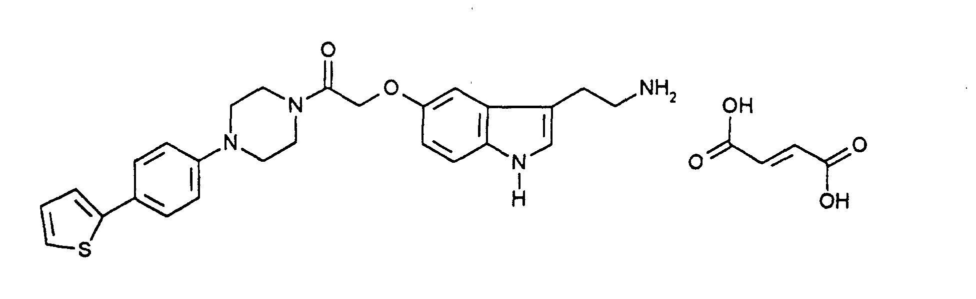

Le composé 10 est préparé à partir de l'intermédiaire 9C (600 mg; 0,96 mmol) et de l'acide 2-thiophène boronique (184 mg; 1,44 mmol) selon la procédure décrite pour la préparation de l'exemple 9.
RMN ¹H DMSO-d6 (ppm) : 2,86 t, 2H; 2,97 t, 2H; 3,18-3,26 m, 4H; 3,60-3,70 m, 4H; 4,81 s, 2H; 6,36 s, 1H; 6,80 dd, 1H; 7,00 d, 2H; 7,05-7,15 m, 2H; 7,16 d, 1H; 7,25 d, 1H; 7,33 d, 1H; 7,40 dd, 1H; 7,51 d, 2H; 10,78 s, 1H

| Analyse élémentaire : C₂₆H₂₈N₄O₂S₁; 0,5C₄H₄O₄, 1H₂O | | | |
|---|---|---|---|
| % Calculés | C 62,27 | H 6,01 | N 10,44 |
| % Trouvés | C 63,03 | H 5,85 | N 10,80 |

Point de fusion : 154-156°C
Spectre de masse : m/z 461 (MH⁺)

### EXEMPLE 11

### Fumarate du 2-[3-(2-amino-éthyl)-1H-indol-5-yloxy]-1-[4-(4-thiophén-3-yl-phényl)-pipérazin-1-yl]-éthanone

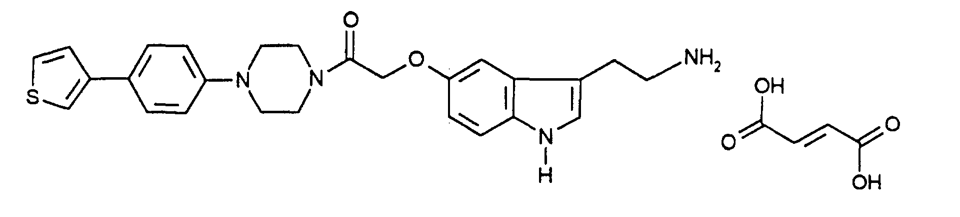

Le composé **11** est préparé à partir de l'intermédiaire **9C** (600 mg; 0,96 mmol) et de l'acide 3-thiophène boronique (184 mg; 1,44 mmol) selon la procédure décrite pour la préparation de l'exemple 9.
RMN ¹H DMSO-d6 (ppm) : 2,90-3,05 m, 4H; 3,10-3,20 m, 4H; 3,65 large s, 4H; 4,82 s, 2H; 6,41 s, 1H; 6,79 dd, 1H; 6,98 d, 2H; 7,15 dd, 2H; 7,25 d, 1H; 7,48 d, 1H; 7,56-7,60 m, 3H; 7,67 d, 1H; 10,82 s, 1H

| Analyse élémentaire : C₂₆H₂₈N₄O₂S₁; 0,6C₄H₄O₄; 1,2H₂O | | | |
|---|---|---|---|
| % Calculés | C 62,22 | H 5,96 | N 10,22 |
| % Trouvés | C 61,88 | H 5,78 | N 9,88 |

Point de fusion : 139-141°C

Les exemples suivants illustrent des compositions selon l'invention. Dans ces exemples, le terme « composant actif» désigne un ou plusieurs (généralement un) des composés de formule (I) selon la présente invention.

### Comprimés

On peut les préparer par compression directe ou en passant par une granulation au mouillé. Le mode opératoire par compression directe est préféré mais il peut ne pas convenir dans tous les cas selon les doses et les propriétés physiques du composant actif.

| *A - Par compression directe* | |
|---|---|
| | mg pour 1 comprimé |
| composant actif | 10,0 |
| cellulose microcristalline B.P.C. | 89,5 |
| stéarate de magnésium | 0,5 |
| | 100,0 |

On passe le composant actif au travers d'un tamis à ouverture de maille de 250 µm de côté, on mélange avec les excipients et on comprime à l'aide de poinçons de 6,0 mm. On peut préparer des comprimés présentant d'autres résistances mécaniques en modifiant le poids de compression avec utilisation de poinçons appropriés.

| *B - Granulation au mouillé* | |
|---|---|
| | mg pour un comprimé |
| composant actif | 10,0 |
| lactose Codex | 74,5 |
| amidon Codex | 10,0 |
| amidon de maïs prégélatinisé Codex | 5,0 |
| stéarate de magnésium | 0,5 |
| Poids à la compression | 100,0 |

On fait passer le composant actif au travers d'un tamis à ouverture de maille de 250 µm et on mélange avec le lactose, l'amidon et l'amidon prégélatinisé. On humidifie les poudres mélangées par de l'eau purifiée, on met à l'état de granulés, on sèche, on tamise et on mélange avec le stéarate de magnésium. Les granulés lubrifiés sont mis en comprimés comme pour les formules par compression directe. On peut appliquer sur les comprimés une pellicule de revêtement au moyen de matières filmogènes appropriées, par exemple la méthylcellulose ou l'hydroxy-propyl-méthyl-cellulose, selon des techniques classiques. On peut également revêtir les comprimés de sucre.

| Capsules | |
|---|---|
| | mg pour une capsule |
| composant actif | 10,0 |
| *amidon 1500 | 89,5 |
| stéarate de magnésium Codex | 0,5 |
| Poids de remplissage | 100,0 |

| | |
|---|---|
| *une forme d'amidon directement compressible provenant de la firme Colorcon Ltd, Orpington, Kent, Royaume Uni. | |

On fait passer le composant actif au travers d'un tamis à ouverture de maille de 250 µm et on mélange avec les autres substances. On introduit le mélange dans des capsules de gélatine dure n°2 sur une machine à remplir appropriée. On peut préparer d'autres unités de dosage en modifiant le poids de remplissage et, lorsque c'est nécessaire, en changeant la dimension de la capsule.

| Sirop | | |
|---|---|---|
| | | mg par dose de 5 ml |
| composant actif | | 10,0 |
| saccharose Codex | | 2750,0 |
| glycérine Codex | | 500,0 |
| tampon | ) | |
| arôme | ) | |
| colorant | ) | q.s. |
| préservateur | ) | |
| eau distillée | | 5,0 |

On dissout le composant actif, le tampon, l'arôme, le colorant et le préservateur dans une partie de l'eau et on ajoute la glycérine. On chauffe le restant de l'eau à 80°C et on y dissout le saccharose puis on refroidit. On combine les deux solutions, on règle le volume et on mélange. Le sirop obtenu est clarifié par filtration.

| Suppositoires | |
|---|---|
| Composant actif | 10,0 mg |
| *Witepsol H15 complément à | 1,0 g |

| | |
|---|---|
| *Marque commercialisée pour Adeps Solidus de la Pharmacopée Européenne. | |

On prépare une suspension du composant actif dans le Witepsol H15 et on l'introduitdans une machine appropriée avec moules à suppositoires de 1 g.

| Liquide pour administration par injection intraveineuse | |
|---|---|
| | g/l |
| composant actif | 2,0 |
| eau pour injection Codex complément à | 1000,0 |

Onpeut ajouter du chlorure de sodium pour régler la tonicité de la solution et régler le pH à la stabilité maximale et/ou pour faciliter la dissolution du composait actif au moyen d'un acide ou d'un alcali dilué ou en ajoutant des sels tampons appropriés. On prépare la solution, on la clarifie et on l'introduit dans des ampoules de dimension appropriée qu'on scelle par fusion du verre. On peut également stériliser le liquide pour injection par chauffage à l'autoclave selon l'un des cycles acceptables. On peut également stériliser la solution par filtration et introduit en ampoule stérile dans des conditions aseptiques. La solution peut être introduite dans les ampoules en atmosphère gazeuse.

| Cartouches pour inhalation | |
|---|---|
| | g/cartouche |
| composant actif micronisé | 1,0 |
| lactose Codex | 39,0 |

Le composant actif est micronisé dans un broyeur à énergie de fluide et mis à l'état de fines particules avant mélange avec du lactose pour comprimés dans un mélangeur à haute énergie. Le mélange pulvérulent est introduit en capsules de gélatine dure n°3 sur une machine à encapsuler appropriée. Le contenu des cartouches est administré à l'aide d'un inhalateur à poudre.

| Aérosolsous pression à valve doseuse | | |
|---|---|---|
| | mg/dose | pour 1 boite |
| composant actif micronisé | 0,500 | 120 mg |
| acide oléique Codex | 0,050 | 12 mg |
| trichlorofluorométhane pour usage pharmaceutique dichlorodifluorométhane | 22,25 | 5,34 g |
| pour usage pharmaceutique | 60,90 | 14,62 g |

Le composant actif est micronisé dans un broyeur à énergie de fluide et mis à l'état de fines particules. On mélange l'acide oléique avec le trichlorofluorométhane à une température de 10-15°C et on introduit dans la solution à l'aide d'un mélangeur à haut effet de cisaillement le médicament micronisé. La suspension est introduite en quantité mesurée dans des boîtes aérosol en aluminium sur lesquelles on fixe des valves doseuses appropriées délivrant une dose de 85 mg de la suspension ; le dichlorodifluorométhane est introduit dans les boites par injection au travers des valves.

### Propriétés pharmacologiques

Les dérivés de la présente invention sont des agonistes très sélectifs des récepteurs 5-HT_{1B/1D} et en particulier des récepteurs 5-HT_{1D} et 5-HT_{1B} humains. L'étude de la liaison de quelques exemples de composés de la présente invention avec ces récepteurs a été réalisée selon la méthode décrite par P. PAUWELS et C. PALMIER (Neuropharmacology, 33, 67, 1994). Cette méthode montre que ces composés ont une affinité inférieure à 100 nM pour les récepteurs humains 5HT_{1D} et 5HT_{1B}. Les dérivés de la présente invention sont en outre capables, comme la sérotonine, d'induire la constriction des anneaux de veine saphène de lapin médiée par les récepteurs 5HT_{1B}. La technique mise en oeuvre a été adaptée de Van Heuven-Nolsen D. et al. (Eur. J. Pharmacol. 191, 375, 1990) et de Martin G.R. et Mc Lennan (Naunyn-Schmideberg's Arch. Pharmacol., 342, 111, 1990).
Ces résultats pharmacologiques illustrent l'intérêt des composés de la présente invention qui sont de nouveaux et puissants agonistes des récepteurs 5-HT_{1B} et 5-HT_{1D}.

En thérapeutique humaine, les composés de formule générale (I) selon l'invention sont particulièrement utiles pour le traitement et la prévention des désordres liés à la sérotonine au niveau du système nerveux central et du système vasculaire. Ces composés peuvent donc être utilisés dans le traitement et la prévention de la dépression, des désordres compulsifs obsessionnels, de la boulimie, de l'agressivité, de l'alcoolisme, du tabagisme, de l'hypertension, de la nausée, du dysfonctionnement sexuel, du comportement asocial, de l'anxiété, de la migraine, de la spasticité, de la maladie d'Alzheimer et des troubles de la mémoire.

## Revendications

1. Composés répondant à la formule générale (I) 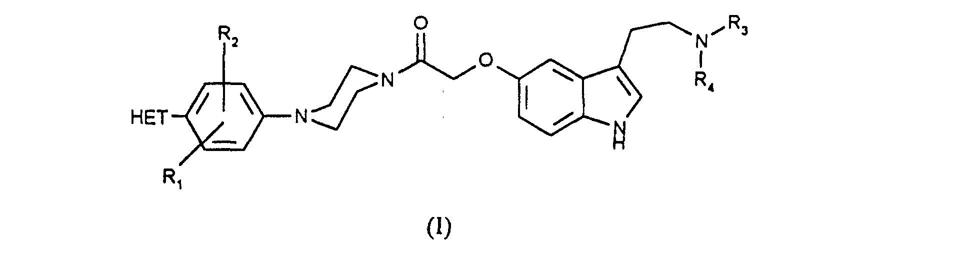 dans laquelle,
HET représente un hétérocycle choisi parmi 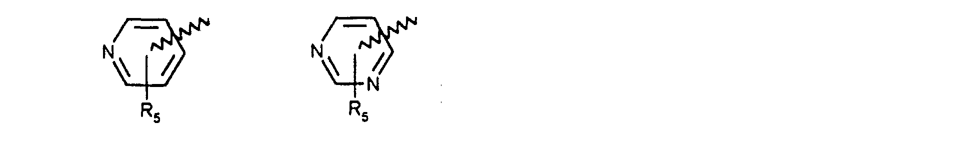 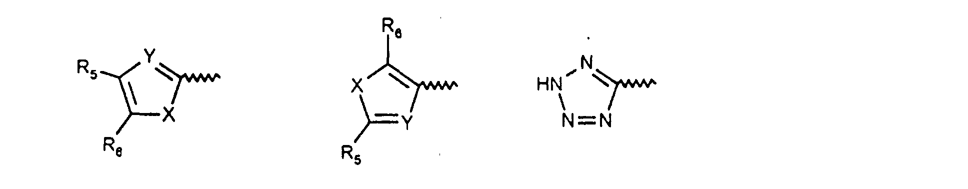 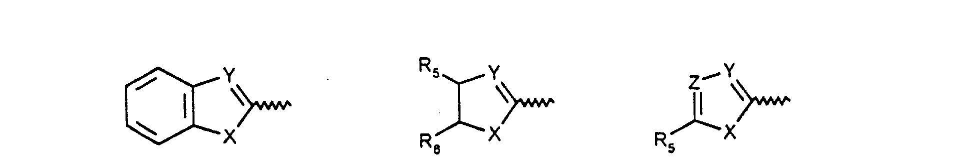
R₁ et R₂, identiques ou différents représentent un hydrogène R'₁, CF₃, CH₂CF₃, C₆H₅, CH₂C₆H₅, OH, OR'₁, SH, SR'₁, Cl, F, Br, I, CN, NH₂, NHR'₁, NR'₁R'₂, NO₂, NH-NH₂, NH-NHR'₁, NHOH, NHCO₂R'₁, NHCONH₂, NHCONR'₁R'₂, NHSO₂R'₁, SO₂R'₁, SO₂NH₂, SO₂NHR'₁, COR'₁, CO₂R'₁, CONH₂, CONHR'₁, CONR'₁R'₂ pouvant être en position ortho ou meta sur le cycle aromatique,
R₃ et R₄, identiques ou différents représentent un hydrogène, un radical carboné linéaire ou ramifié comprenant de 1 à 6 atomes de carbone ou un reste benzyle ou phénétyle,
Y et Z, identiques ou différents représentent CH ou N,
X représente O, S ou NR₇,
R₅, R₆ et R₇, identiques ou différents, représentent un hydrogène, un reste alkyle linéaire ou ramifié comprenant de 1 à 6 atomes de carbone ou un reste phényle éventuellement substitué par un reste alkyle linéaire ou ramifié comprenant de 1 à 6 atomes de carbone, un halogène, CF₃, OCH₃, CN, ou NO_{2,}
R'₁ et R'₂, identiques ou différents, représentent un groupement alkyle linéaire ou ramifié comprenant de 1 à 6 atomes de carbone ou un reste phényle éventuellement substitué par un reste alkyle linéaire ou ramifié comprenant de 1 à 6 atomes de carbone, Cl, Br, F, I, OCH₃, OH, NO₂, SCH₃,
ainsi que leurs sels et solvats acceptables pour l'usage thérapeutique.

2. Composés selon la revendication 1, **caractérisés en ce que** R₁, R₂, R₃ et R₄ représentent chacun un hydrogène.

3. Composés selon la revendication 1 ou 2, **caractérisés en ce que** HET représente un reste pyridyle ou pyrimidyle.

4. Composés selon la revendication 1 ou 2, **caractérisés en ce que** HET représente un hétérocycle à 5 chaînons contenant de 1 à 3 hétéroatomes choisis parmi O, S ou N.

5. Composés selon l'une des revendications 1 à 4 à l'état de sels acceptables pour l'usage thérapeutique, **caractérisés en ce que** ces sels sont choisis parmi les chlorhydrates, les bromhydrates, les sulfates, les fumarates, les maléates, les méthanesulfonates et les succinates.

6. Procédé de préparation des composés de formule générale (I) selon la revendication 1 **caractérisé en ce que** l'on fait réagir un intermédiaire de formule générale (II) 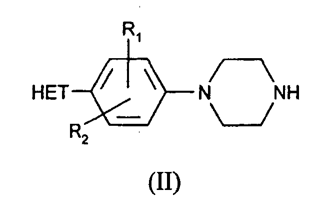 dans laquelle HET, R₁ et R₂ sont définis comme dans la formule générale (I) avec un acide carboxylique ou un dérivé d'acide carboxylique de formule générale (III) 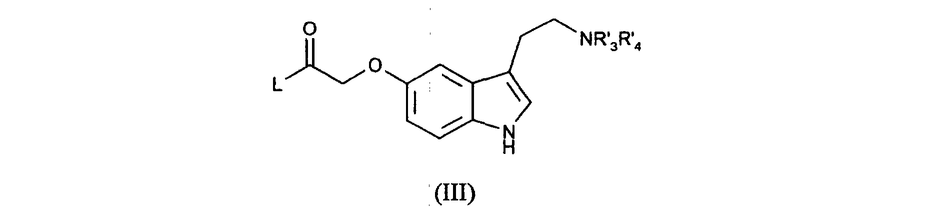 dans laquelle R'₃ et R'₄ sont identiques à R₃ et R₄ tels que définis dans la formule générale (I), ou R'₃ et R'₄ sont des précurseurs ou groupes protecteurs de R₃ et R₄ qui seront transformés en R₃ et R₄ à la suite de la condensation de (II) avec (III), et L représente OH, Cl, O-alkyle ou le groupe -C(=O)L représente la forme activée d'un acide carboxylique.

7. Composés selon l'une des revendications 1 à 5 pour leur application en tant que substances thérapeutiquement actives.

8. Composés selon la revendication 7 pour le traitement ou la prévention des désordres liés à la sérotonine.

9. Composés selon la revendication 7 pour le traitement ou la prévention de la migraine, de l'algie vasculaire de la face et des céphalées chroniques vasculaires.

10. Composés selon la revendication 7 pour le traitement ou la prévention de la dépression, des désordres compulsifs obsessionnels, de la boulimie, de l'agressivité, de l'alcoolisme, des nausées, du dysfonctionnement sexuel, du comportement asocial, de l'anxiété, de la spasticité, des maladies d'Alzheimer et de Parkinson.

11. Compositions pharmaceutiques contenant à titre d'ingrédient actif au moins un composé selon l'une des revendications 7 à 10 et un excipient pharmaceutiquement acceptable.

## Patentansprüche

1. Verbindungen, welcher der allgemeinen Formel (I) entsprechen 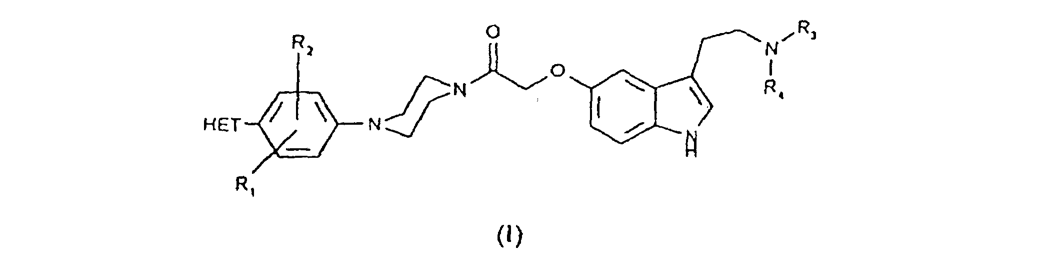 in welcher:
HET steht für einen Heterocyclus, welcher ausgewählt wird unter 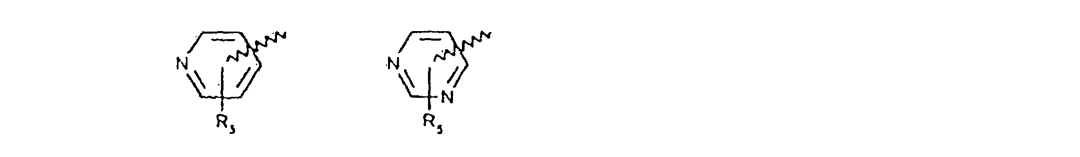 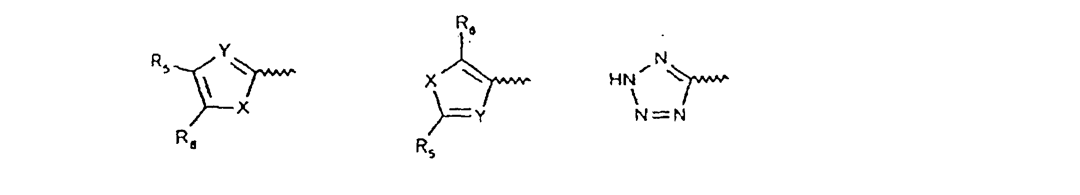 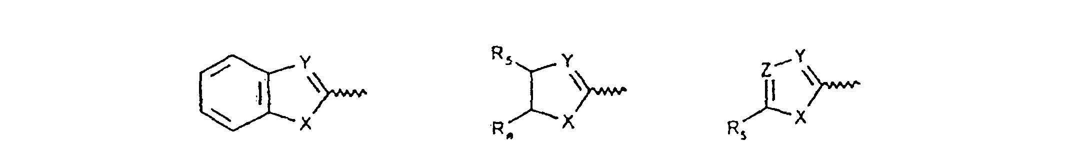
R₁ und R₂, welche gleich oder verschieden sind, stehen für ein Wasserstoffatom, R'₁, CF₃, CH₂CF₃, C₆H₅, CH₂C₆H₅, OH, OR'₁, SH, SR'₁, Cl, F, Br, I, CN, NH₂, NHR'₁, NR'₁R'₂, NO₂, NH-NH₂, NH-NHR'₁, NHOH, NHCO₂R'₁, NHCONH₂, NHCONR'₁R'₂, NHSO₂R'₁, SO₂R'₁, SO₂NH₂, SO₂NHR'₁, COR'₁, CO₂R'₁, CONH₂, CONHR'₁, CONR'₁R'₂, welche sich in ortho- oder meta-Position an dem aromatischen Cyclus befinden können,
R₃ und R₄, welche gleich oder verschieden sind, stehen für ein Wasserstoffatom, einen linearen oder verzweigten kohlenstoffhaltigen Rest, welcher 1 bis 6 Kohlenstoffatome umfasst, oder einen Benzyl- oder Phenethylrest,
Y und Z, welche gleich oder verschieden sind, stehen für CH oder N,
X steht für O, S oder NR₇,
R₅, R₆ und R₇, die gleich oder verschieden sind, stehen für ein Wasserstoffatom, einen linearen oder verzweigten Alkylrest, welcher 1 bis 6 Kohlenstoffatome umfasst, oder einen Phenylrest, welcher gegebenenfalls substituiert ist durch einen linearen oder verzweigten Alkylrest, welcher 1 bis 6 Kohlenstoffatome umfasst, ein Halogen, CF₃, OCH₃, CN oder NO₂,
R'₁ und R'₂, welche gleich oder verschieden sind, stehen für eine lineare oder verzweigte Alkylgruppe, welche 1 bis 6 Kohlenstoffatome umfasst, oder einen Phenylrest, welcher gegebenenfalls substituiert ist durch einen linearen oder verzweigten Alkylrest, welcher 1 bis 6 Kohlenstoffatome umfasst, Cl, Br, F, I, OCH₃, OH, NO₂, SCH₃.

2. Verbindungen nach Anspruch 1, **dadurch gekennzeichnet, dass** R₁, R₂, R₃ und R₄ jeweils für ein Wasserstoffatom stehen.

3. Verbindungen nach Anspruch 1 oder 2, **dadurch gekennzeichnet, dass** HET für einen Pyridyl- oder Pyrimidylrest steht.

4. Verbindungen nach Anspruch 1 oder 2, **dadurch gekennzeichnet, dass** HET für einen Heterocyclus mit 5 Ringgliedern, welcher 1 bis 3 Heteroatome, ausgewählt unter O, S oder N, enthält, steht.

5. Verbindungen nach einem der Ansprüche 1 bis 4 im Zustand von Salzen, welche für die therapeutische Verwendung annehmbar sind, **dadurch gekennzeichnet, dass** diese Salze unter den Hydrochloriden, den Hydrobromiden, den Sulfaten, den Fumaraten, den Maleaten, den Methansulfonaten und den Succinaten ausgewählt werden.

6. Verfahren zur Herstellung der Verbindungen der allgemeinen Formel (I) nach Anspruch 1, **dadurch gekennzeichnet, dass** man ein Zwischenprodukt der allgemeinen Formel (II) 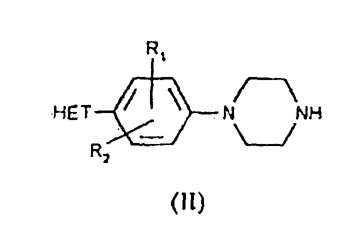 in welcher HET, R₁ und R₂ wie in der allgemeinen Formel (I) definiert sind, reagieren lässt mit einer Carbonsäure oder einem Carbonsäurederivat der allgemeinen Formel (III), 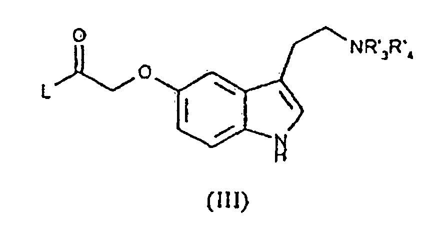 in welcher R'₃ und R'₄ identisch sind mit R₃ und R₄, wie in der allgemeinen Formel (I) definiert, oder R'₃ und R'₄ Vorstufen oder Schutzgruppen von R₃ und R₄, die nach der Kondensation von (II) mit (III) in R₃ und R₄ umgewandelt werden, sind, und L für OH, Cl, O-Alkyl oder die Gruppe -C(=O)L, die aktivierte Form einer Carbonsäure darstellt, steht.

7. Verbindungen nach einem der Ansprüche 1 bis 5 für deren Anwendung als therapeutisch wirksame Substanzen.

8. Verbindungen nach Anspruch 7 für die Behandlung oder die Verhütung von Erkrankungen oder Störungen, welche mit Serotonin verbunden sind.

9. Verbindungen nach Anspruch 7 für die Behandlung oder Verhütung von Migräne, vaskulären Algien des Gesichts und chronischen vaskulären Kopfschmerzen.

10. Verbindungen nach Anspruch 7 für die Behandlung oder Verhütung von Depressionen, obsessiv-kompulsiven Erkrankungen oder Störungen, Bulimie, Aggressivität, Alkoholismus, Übelkeit, sexuellen Dysfunktionen, asozialem Verhalten, Angst, Spastizität, Alzheimer'- und Parkinson'-Krankheit.

11. Pharmazeutische Zusammensetzungen, welche als aktiven Bestandteil wenigstens eine Verbindung nach einem der Ansprüche 7 bis 10 und eine pharmazeutisch annehmbare Trägersubstanz enthalten.

## Claims

1. Compounds corresponding to general formula (I) 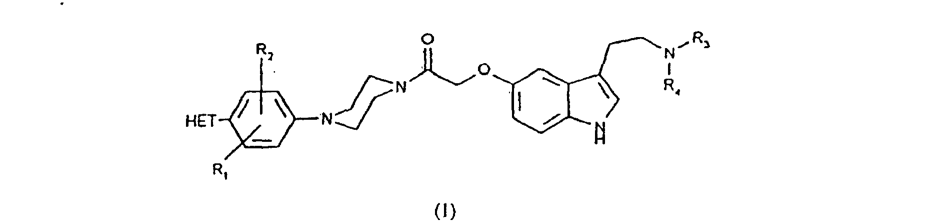 in which,
HET represents a heterocycle chosen from 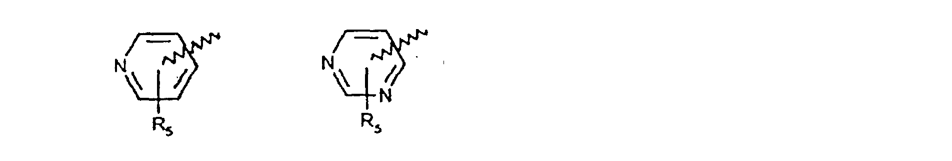 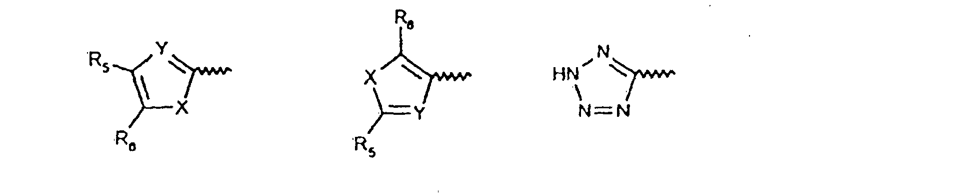 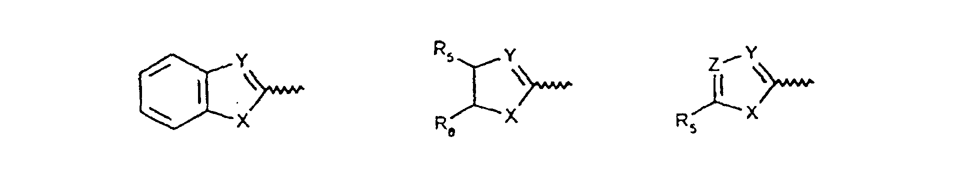
R₁ and R₂, which are identical or different, represent a hydrogen atom, R'₁, CF₃, CH₂CF₃, C₆H₅, CH₂C₆H₅, OH, OR'₁, SH, SR'₁, Cl. F, Br, I, CN, NH₂, NHR'₁, NR'₁R'₂, NO₂, NH-NH₂, NH-NHR'₁, NHOH, NHCO₂R'₁, NHCONH₂, NHCONR'₁R'₂, NHSO₂R'₁, SO₂R'₁, SO₂NH₂, SO₂NHR'₁, COR'₁, CO₂R'₁, CONH₂, CONHR'₁, CONR'₁R'₂ which may be at the ortho or meta position on the aromatic ring,
R₃ and R₄, which are identical or different, represent a hydrogen atom, a linear or branched carbon-containing radical comprising from 1 to 6 carbon atoms or a benzyl or phenethyl residue,
Y and Z, which are identical or different, represent CH or N,
X represents O, S or NR₇,
R₅, R₆ and R₇, which are identical or different, represent a hydrogen atom, a linear or branched alkyl residue comprising from 1 to 6 carbon atoms or a phenyl residue which is optionally substituted with a linear or branched alkyl residue comprising from 1 to 6 carbon atoms, a halogen atom, CF₃, OCH₃, CN or NO₂,
R'₁ and R'₂, which are identical or different, represent a linear or branched alkyl group comprising from 1 to 6 carbon atoms or a phenyl residue which is optionally substituted with a linear or branched alkyl residue comprising from 1 to 6 carbon atoms, Cl, Br, F, I, OCH₃, OH, NO₂, SCH₃,
as well as their salts and solvates which are acceptable for therapeutic use.

2. Compounds according to claim 1, **characterized in that** R₁, R₂, R₃ and R₄ each represent a hydrogen atom.

3. Compounds according to claim 1 or 2, **characterized in that** HET represents a pyridyl or pyrimidyl residue.

4. Compounds according to claim 1 or 2, **characterized in that** HET represents a 5-membered heterocycle containing from 1 to 3 heteroatoms chosen from O, S or N.

5. Compounds according to one of claims 1 to 4, in the form of salts which are acceptable for therapeutic use, **characterized in that** these salts are chosen from the hydrochlorides, hydrobromides, sulphates, fumarates, maleates, methanesulphonates and succinates.

6. Method of preparing the compounds of general formula (I) according to claim 1, **characterized in that** an intermediate of general formula (II) 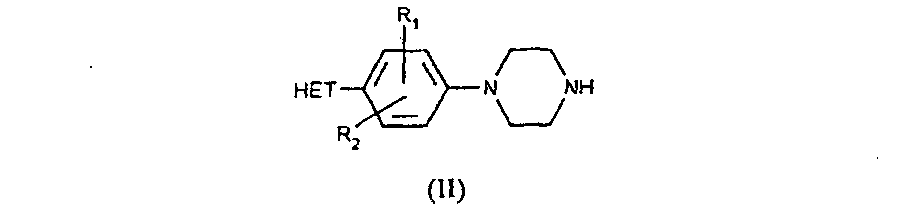 in which HET, R₁ and R₂ are defined as in general formula (I) is reacted with a carboxylic acid or a carboxylic acid derivative of general formula (III) 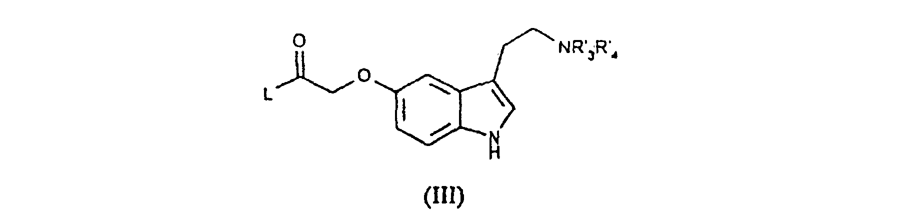 in which R'₃ and R'₄ are identical to R₃ and R₄ as defined in general formula (I), or R'₃ and R'₄ are precursors of or protecting groups for R₃ and R₄ which will be converted to R₃ and R₄ following the condensation of (II) with (III), and L represents OH, Cl, O-alkyl or the group -C(=O)L represents the activated form of a carboxylic acid.

7. Compounds according to one of claims 1 to 5, for their application as therapeutically active substances.

8. Compounds according to claim 7, for the treatment or prevention of serotonin-related disorders.

9. Compounds according to claim 7, for the treatment or prevention of migraine, vascular facial pain and chronic vascular cephalalgia.

10. Compounds according to claim 7, for the treatment or prevention of depression, obsessive-compulsive disorders, bulimia, aggressiveness, alcoholism, nausea, sexual dysfunction, antisocial behavior, anxiety, spasticity, Alzheimer's and Parkinson's diseases.

11. Pharmaceutical compositions containing, as active ingredient, at least one compound according to one of claims 7 to 10 and a pharmaceutically acceptable excipient.
